# EUROPEAN PATENT APPLICATION

(11) **EP 3 940 063 A2**
(43) Date of publication of application: **19.01.2022**
(21) Application number: 20757209.0
(22) Date of filing: 09.03.2020
(51) Int. Cl.: C12N 5/0783, A61K 39/00, C07K 14/55

(54) **METHOD FOR THE EXPANSION AND DIFFERENTIATION OF T LYMPHOCYTES AND NK CELLS IN ADOPTIVE TRANSFER THERAPIES**

(30) Priority: 15.03.2019 CU 20190021
(71) Applicant: Centro de Inmunologia Molecular, La Habana 11600 (CU); Ludwig Institute for Cancer Research Ltd, 8001 Zürich (CH); Centre Hospitalier Universitaire Vaudois, 1011 Lausanne (CH); University of Lausanne, 1015 Lausanne (CH)
(72) Inventor: LEÓN MONZÓN, Kalet, Habana 17100 (CU); MONTALVO BEREAU, Galia Magela, La Habana 10700 (CU); COUKOS, George, 1011 Lausanne (CH); IRVING, Melita, 1066 Epalinges (CH); CRIBIOLI, Elisabetta, VD 1066 Epalinges (CH); ORTIZ MIRANDA, Yaquelín, Camagüey 72810 (CU); CORRIA OSORIO, Ángel de Jesús, VD 1066 Epalinges (CH)
(74) Representative: V.O.
(86) International application number: PCT/CU2020/050002
(87) International publication number: WO 2020/187340

(57) **Abstract**

The present invention describes a method for obtaining lymphoid cells having a desired phenotype for adoptive transfer therapies useful for the treatment of cancer. Especially, this invention is related to strategies for inducing preferential signaling through the intermediate affinity IL-2 receptor in order to expand the cells with a desired central memory phenotype. The method of the present invention is useful for obtaining tumor-infiltrating lymphocytes, TCR or chimeric antigen receptor engineered T cells for the treatment of cancer.

SEQUENCE LISTING

<110>  Centro de Inmunología Molecular
       LUDWIG INSTITUTE FOR CANCER RESEARCH LTD
       CENTRE HOSPITALIER UNIVERSITAIRE VAUDOIS
       UNIVERSITY OF LAUSANNE

<120>  Method for the expansion and differentiation of T lymphocytes and NK
cells in adoptive transfer therapies.

<130>  602018CU00

<160>  7

<170>  PatentIn version 3.5

<210>  1
<211>  133
<212>  PRT
<213>  Artificial Sequence

<220>
<223>  by DNA recombinant technology

<400>  1

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Lys Met Leu Thr Ile Lys Phe Asn Met Pro Lys
        35                  40                  45

Lys Ala Thr Glu Leu Lys His Leu Gln Cys Leu Glu Glu Leu Leu Lys
    50                  55                  60

Pro Leu Glu Val Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130

<210>  2
<211>  133
<212>  PRT
<213>  Artificial Sequence

<220>
<223>  by DNA recombinant technology

<400>  2

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Lys Met Leu Thr Gln Lys Phe Glu Met Pro Lys
        35                  40                  45

Lys Ala Thr Glu Leu Lys His Leu Gln Cys Leu Glu Glu Glu Leu Lys
    50                  55                  60

Pro Leu Glu Val Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130


<210>  3
<211>  133
<212>  PRT
<213>  Artificial Sequence

<220>
<223>  by DNA recombinant technology

<400>  3

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Ala Met Leu Thr Ile Lys Phe Asn Met Pro Lys
        35                  40                  45

Lys Ala Thr Glu Leu Lys His Leu Gln Cys Leu Glu Glu Leu Leu Lys
    50                  55                  60

Pro Leu Glu Val Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130

<210>  4
<211>  133
<212>  PRT
<213>  Artificial Sequence

<220>
<223>  by DNA recombinant technology

<400>  4

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Lys Met Leu Thr Lys Lys Phe Arg Met Pro Lys
        35                  40                  45

Lys Ala Thr Glu Leu Lys His Leu Gln Cys Leu Glu Glu Leu Leu Lys
    50                  55                  60

Pro Leu Glu Val Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130

<210>  5
<211>  133
<212>  PRT
<213>  Artificial Sequence

<220>
<223>  by DNA recombinant technology

<400>  5

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Lys Met Leu Thr Ile Lys Phe Glu Met Pro Lys
        35                  40                  45

Lys Ala Thr Glu Leu Lys His Leu Gln Cys Leu Glu Glu Glu Leu Lys
    50                  55                  60

Pro Leu Glu Val Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130

<210>  6
<211>  133
<212>  PRT
<213>  Artificial Sequence

<220>
<223>  by DNA recombinant technology

<400>  6

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Ala Met Leu Thr Ala Lys Phe Ala Met Pro Lys
        35                  40                  45

Lys Ala Thr Glu Leu Lys His Leu Gln Cys Leu Glu Glu Ala Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130


<210>  7
<211>  133
<212>  PRT
<213>  Artificial Sequence

<220>
<223>  by DNA recombinant technology

<400>  7

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Lys Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr
    130

## Description

### FIELD OF THE TECHNIQUE

The present invention relates to the field of Biotechnology and immune-oncology. It is related to methods for obtaining lymphoid cells with a desired central memory phenotype and their use for adoptive cell transfer therapies in cancer patients.

### BACKGROWND

Interleukin-2 (IL2) was first cytokines discovered to be molecularly characterized. It was primarily shown to support the growth and expansion of T and NK cells (Morgan et. al., 1976, Science, 193 (4257):1007-1008). IL2 was approved for clinical use in 1992, but the precise description of the biology of its receptor is still under study (Rosenberg, 2014, J. Immunol., 192(12):5451-8; Smith, 2006, Medical Immunology, 1476 (9433):5-3). IL2 administration at high doses (HD IL2) is used as a cancer immunotherapy. Systemic HD IL2 treatment produces durable responses in melanoma and renal cancer carcinoma patients, but only in a relatively small fraction of patients. Moreover, systemic HD IL2 treatments induce significant toxicities further limiting its clinical relevance (Atkins, 2006, Clin. Cancer Res., 12:2353-2358).

IL2 interacts with three types of receptors (IL2Rs) on lymphoid cells: The low affinity receptor (Kd -10-8 M), which contains only the IL2Rα subunit (CD25); The intermediate affinity receptor (Kd -10-9 M), which is formed by the IL2Rβ and IL2Rγ subunits (CD122 and CD132); and the high affinity receptor (Kd ~10-11 M), which contains the three subunits IL2Rα, IL2Rβ and IL2Rγ. Only the intermediate-affinity (IL2Rβγ) and the high-affinity (IL2Rαβγ) receptors are functional, being capable of transducing signals to the cells (Stauber et. al., 2005, PNAS, 103(8):2788-2793; Wang, 2005, Science, 310(18): 1159-1163).

IL2Rβ and IL2Rγ subunits are present on effector lymphoid cells of the immune system, but the constitutive expression of the IL2Ra subunit gives to regulatory T cells (Tregs) the high affinity receptor and therefore a preferential use of IL2 in vivo (Malek, 2008, Annu. Rev. Immunol., 26:453-79). Nowadays, it is known that part of the limitations to the efficacy of systemic HD IL2 therapy in cancer patients is due to the preferential IL2-driven expansion of Tregs, which, in turn, dampens antitumor immunity (Choo Sim et. al., 2014, J. Clin. Invest., 124(1):99-110)

Many attempts have been made to improve the efficacy of systemic HD IL2 therapy for cancer. Several studies have shown that it is possible to mutate native IL2 to either increase or reduce some of its biologic properties, based on the differential expression of IL2 receptor subunits on lymphoid cells (Skrombolas and Frelinger, 2014, Expert. Rev. Clin. Immunol., 10(2):207-217)). Initial studies focused in enhancing IL2 affinity for the IL2Ra (Rao et al., 2003, Protein Engineering, 16(12):1081-1087). Although not evident at the time, it is now appreciated that this strategy actually downregulate immune response *in vivo* as a consequence of massive stimulation of Tregs. More recently, Levin et. al. in 1992 obtained IL2 mutants, with enhanced binding to the IL2Rβ subunit, which preferentially activate effector cells overexpressing the intermediate affinity IL2 receptor (Levin et. al., 2012, Nature, 484 (7395):529-33). This mutein termed "superkine H9" showed improved in vivo antitumoral effect and less toxicity than native IL2. Carmenate *et al* reported the introduction of four mutations into human IL2, which efficiently reduced the affinity for the IL2Rα without affecting the interaction with the dimeric intermediate affinity receptor (IL2Rβγ). This mutein avoided Tregs expansion when administered in vivo and resulted in more effective and less toxic than native IL2 in mouse models (Carmenate et. al., 2013, J. Immunol., 190(12): 6230-8). Two other IL2 muteins, namely F42K and R38A have also greatly decrease IL2 binding affinity to the IL2Rα and showed less effect in stimulating Tregs in vitro while effectively expands LAK cells (Heaton et. al., 1994, Ann. Surg. Oncol., 1(3):198-203).

In another attempt to improve systemic HD IL2 therapy, several works focused in increasing half life time by its PEGylation or its fusion to the Fc portion of antibodies (Ab). Indeed, some of these strategies resulted in an effective increase of IL2 lifetime *in vivo* with a positive impact on the overall antitumoral effect (Zhu et. al., 2015; Cancer Cell, 27:498-501; Charych et. al., 2016, Clin. Cancer Res., 22(3):680-90).

Adoptive cell transfer (ACT) is defined as a treatment method in which cells are recovered from a donor, cultured and/or manipulated in vitro, and then administered to a patient for the treatment of a disease. For the treatment of cancer, ACT frequently involves the transfer of lymphocytes (Gattinoni et. al., 2006, Nat. Rev. Immun., 6:383-393).Three main treatment modalities prevail today: a) the use of ex vivo expanded tumor infiltrating lymphocytes (TILs); b) the use of T or NK cells genetically engineered to express a T cell receptor (TCR) with high affinity/avidity for tumor-antigens derived peptides; and c) the use of T or NK cells engineered to express a chimeric antigen receptor (CAR) specific for tumor-antigens. Frequently, the latter strategies are combined with further genetically engineering of the transferred lymphocytes to enhance their effector functions in vivo; for instance, engineering the secretion of relevant cytokines as IL12 and others (Cassian Y., 2018, Curr. Opin. Immunol., 51:197-203).

Despite their capacity to induce antitumor immunity, the broad application of ACT to treat cancer has several well-known limitations. Although transfer of tumor-reactive T effector cells can cause objective responses, there is strong evidence in both human and mice demonstrating that T cells with a central memory phenotype are superior mediators of antitumoral responses (Klebanoff et al., 2005, PNAS, 102(27):9571-6). Central memory T cells are antigen-experienced cells (CD44+) that express CD62L molecule, necessary for migration to peripheral lymph nodes (Ridell et. al., 2014, Cancer J., 20(2):141-144). They persist longer *in vivo*, most likely due to their greater capacity to proliferate and secrete cytokines, like IL2, upon antigen re-encounter (TCF1+). By contrast, effector memory T cells are antigen-experienced cells with significantly downregulation of CD62L molecule, upregulation of inhibitory receptors (PD1, TIM3, LAG3), and impairment of cytokine release. They have a propensity to progressively get exhausted in vivo (Kishton et. al. 2017, Cell. Metab., 26(1):94-109; Klebanoff et. al., 2005, PNAS, 102(27):9571-6). Therefore, culture conditions, which promote the expansion of cells with a central memory phenotype, are much desired for developing better ACT cancer immunotherapies.

IL2 promotes the activation and expansion of T cells and NK cells *in vitro*, therefore it has been a major player in the development of ACT therapies for cancer. In early strategies, IL2 was used to generate lymphokine activated killer (LAK) cells. Co-administration of LAK with systemic HD IL2 increased the clinical response of the systemic monotherapy. In other approach, a high concentration of IL2 is used to primarily expand tumor infiltrating lymphocytes (TILs) from tumor extracts. TILs undergo further rapid expansion in the presence of IL2 combined with TCR stimulation in vitro. Expanded TILs are infused into the cancer patients, together with a systemic HD IL2 regime. The systemic HD IL2 regime contributes to sustain the in vivo persistence of the transferred cells. As overall result, the treatment with TILs + HD IL2 produced some impressive large tumors regressions, but retained the known limitations of systemic HD IL2-therapy (Rosenberg, 2014, J. Immunol., 192 (12) 5451-8). A more recent attempt to increase the efficacy of systemic HD IL2, as an adjuvant for ACT, developed a method based on receptor-ligand orthogonalization, using a mutant IL2 cytokine and mutant IL2 receptor that bind specifically to one another but not to their wild-type human counterparts. With this strategy, the authors redirect the specificity of IL2 toward the engineered T cells using orthogonal IL-2 cytokine-receptor pairs, for the selective expansion of the engineered transferred T cells but with limited off-target activity and negligible toxicity. Ortho-IL2R T cells expanded in orthoIL2 were effective in reducing tumor volume and increasing survival when used in a mouse cancer model of ACT (Sockolosky et. al., 2018, Science, 359 (6379):1037-1042).

Despite the unique and effective role of IL2 for modulating T cell responses associated to ACT, the precise effect of IL2 (positive or negative) on the expansion/differentiation of memory T cells is not completely understood. Cells expanded *in vitro* in the presence of IL2 exhibit a strong effector capacity, which also shorten their long term persistence and survival in vivo. IL2 promotes activation and proliferation of T cells in a unique manner, however also induces activation-induced cell death (Spolski et. al., 2018, Nat. Rev. Immunol., (18) 648-659). Studies have shown that reducing IL2 signaling during in vitro priming, could favor the development of memory CD8+ T cells. Reduction of IL2 signaling has been mainly achieved by lowering IL2 concentration or reducing exposure time during the *in vitro* culture. Other authors partially substitute the use of IL2 with other cytokines from the common γ chain family. Many strategies for vitro T cell activation and expansion in vitro use IL7, IL15 or IL21 in different protocols, but typically combined with some IL2 (Hinrichs et .al., 2008; Blood, 111(11) 5326-5333; Mueller et. al., 2008, Eur. J. Immunol., 38(10) 2874-85; Zeng et. al., 2005, J. Exp. Med., 201(1) 139-48; Markley and Sadelain, 2010, Blood, 115(17) 3508-3519; Zhang et. al., 2015, Clin. Cancer Res., 21(10): 2278-88). Therefore, IL2 remains as a standard for T cells activation and expansion in ACT immunotherapies.

Interestingly, the complexity of IL2/IL2R interaction and its implications on T cells differentiation remains unclear. Particularly, the impact of a preferential signaling through different IL2R (high vs intermediate affinity), on lymphoid cells expansion/differentiation in vitro is still unknown. None of the above-mentioned IL2 muteins has been used to expand and differentiate T cells in vitro for ACT, neither have been used strategies of rationally modifying IL2/IL2R interaction to preferentially direct IL2 signaling through one of the well-known functional forms of the IL2R.

The inventors of the present application have surprisingly found a substantial advantage when using different agents/strategies, which by disrupting IL2/IL2Rα interaction during cells activation/expansion in vitro, preferentially direct IL2 signal through the intermediate affinity IL2R. Such agents/strategies induce an efficient expansion of tumor-specific cells with marked central memory phenotype and a large antigen specific killing capacity as compared to the traditional culturing strategies, which use native IL2 signaling at some point. The present invention allows for a substantial improvement of the current protocols for cell expansion/differentiation in vitro, before its use on adoptive transfer therapies in cancer patients.

### BRIEF DESCRIPTION OF THE INVENTION

The present invention provides an *in vitro* or *ex vivo* method for enrichment and expansion of lymphocytes with a central memory phenotype comprising expanding lymphocytes in a sample obtained from a subject or lymphocytes isolated from such sample, wherein expanding comprises the activation of the β/γ dimeric IL-2 receptor.

The present invention provides an *in vitro* or *ex vivo* method for enrichment and expansion of lymphocytes with a central memory phenotype comprising expanding lymphocytes in a sample obtained from a subject or lymphocytes isolated from such sample, wherein expanding comprises the activation of the β/γ dimeric IL-2 receptor.

The present invention provides a method for enrichment and expansion of lymphocytes with a central memory phenotype for use in adoptive cell transfer therapies, wherein expanding comprises the activation of the β/γ dimeric IL-2 receptor.

The present invention is related to a method for expansion and/or differentiation of lymphoid cell populations with a central memory phenotype useful in adoptive cell transfer therapies that comprise three stages. Said stages are:
i) extraction of lymphoid cells from a subject,
ii) *in vitro* expansion of the lymphoid cells or optionally the lymphoid cells, which have been further genetically engineered, while inducing a preferential signaling through the intermediate affinity IL-2 receptor (referred bellow as IL2Rβγ-biased-IL2 signaling) and
iii) transfer of the activated/expanded cells into a subject with cancer.

Particularly, this method employs different strategies for inducing preferential signaling through the intermediate affinity IL2 receptor (providing IL2Rβγ-biased-IL2 signaling) while activating/expanding the lymphoid cells. These strategies could be any of the followings:
- Culturing the lymphoid cells with a soluble IL-2 mutein, which preferentially signal though the intermediate affinity IL2R.
- Genetic modification of the lymphoid cells for secreting IL2 muteins, which preferentially signals though the intermediate affinity IL2R.
- culturing the lymphoid cells with native IL-2, but:
   a) Adding a pharmacologic agent, which block IL2-IL2Rα interaction or,
   b) Genetically engineering the lymphoid cells for secreting a soluble protein, which block the IL2-IL2Rainteraction or,
   c) Genetically engineering the lymphoid cells to decrease the expression of the IL2Rα receptor subunit (CD25) at the cell surface or,
   d) Genetically engineering the lymphoid cells to increase the expression of the intermediate affinity IL-2 receptor at the cell surface.

More particularly:
The IL-2 muteins used in the method comprises those described in US 9,206,243 which correspond to SEQ ID NO. 1-6 of the present invention; the Superkine H9 disclosed by Levin et al. in 2012, which corresponds to SEQ ID NO. 7 of the present application as well as the F42 mutein described in SEQ ID NO 1 of WO 2017/202786 which corresponds to SEQ ID NO. 8 of the present invention. But without been limited to these molecules (Levin et al. in: Nature (2012). 48: 529-535).

The pharmacologic agents used, which block the IL2-IL2Rα interaction, are selected from: an antibody or antibody fragment, which bind either to IL2Rα or to IL2; a peptide or a chemically defined small molecule, which bind either to IL2Rα or to IL2; a soluble form of the IL2Rα (CD25) or a modified variant of it. But without being limited to them.

In stage ii of the method the IL2Rβγ-biased-IL2 signaling could be provided in different moments of lymphoid cells culturing/expansion. IL2Rβγ-biased-IL2 signaling could be provided all the time or just part of the time during the *in vitro* culture. Moreover, IL2Rβγ-biased-IL2 signaling could or could not be maintained after the *in vivo* transference of cells. In stage ii the lymphoid cell expansion could or could not be done in the presence of other cytokines, for instances IL12, IL17, IL15 and IL21.

In stage iii, the cell transference could be done into the donor subject of stage i or into a different one.

In some embodiments, the expansion of lymphocytes with a central memory phenotype comprises the use of additional hormones, cytokines, and culturing conditions optimized for the lymphocytes expansion.

Lymphoid cells subject to the methods could be any of the followings: a mixture of T cells purified CD4 of CD8 T cells, NK cells, NKT cells. Lymphoid cells could be obtained in stage i from peripheral blood mononuclear cells, tumor draining lymph nodes or tumor infiltrates.

Lymphoid cells in the methods could or could not have been further engineered to: express a CAR; express a TCR of desired specificity; Express other receptors of interest in the cell membrane; Secrete different cytokines or soluble proteins of interest.

Overall, the method of the present invention allows prolonging the persistence of the transferred cells and a higher anti-tumoral effect *ex vivo*, *in vitro* and *in vivo.*

In certain embodiments, the sample is obtained from draining lymph nodes. In other embodiments, the sample is an untreated tumor fragment, enzymatically treated tumor fragment, dissociated/suspended tumor cells, a lymph node sample, or a bodily fluid (e.g., blood, ascites, or lymph) sample.

In certain embodiments, the subject is human.

In another aspect, described herein are methods of treating a tumor in a subject in need thereof comprising administering to the subject an effective amount of the lymphocytes made by the methods as disclosed herein. In certain embodiments, the tumor is a solid tumor. In certain embodiments, the tumor is a liquid tumor.

Another subject of the present invention is the use of the method in adoptive cell transfer therapy, particularly in obtaining tumor-infiltrating lymphocytes or chimeric antigen receptor T and more particularly in the treatment of cancer.

The cells obtained by method of the present invention are also subject of the present invention.

### DETAILED DESCRIPTION OF THE INVENTION

The present invention provides an *ex vivo* method for enrichment and expansion of lymphocytes with a central memory phenotype comprising expanding lymphocytes in a sample obtained from a subject or lymphocytes isolated from such sample, wherein expanding comprises the activation of the β/γ dimeric IL-2 receptor.

The present invention provides an *in vitro* method for enrichment and expansion of lymphocytes with a central memory phenotype comprising expanding lymphocytes in a sample obtained from a subject or lymphocytes isolated from such sample, wherein expanding comprises the activation of the β/γ dimeric IL-2 receptor.

The present invention provides a method for enrichment and expansion of lymphocytes with a central memory phenotype for use in adoptive cell transfer therapies, wherein expanding comprises the activation of the βγ dimeric IL-2 receptor.

The present invention relies on altering the signaling through the IL2 receptor on T lymphocytes, *ex vivo*, *in vitro* and *in vivo*, for its use in ACT therapy. This invention propose primarily to substitute the native IL2 (and its native derived signal) used for the *in vitro* activation and expansion of lymphoid cells, before their transfer *in vivo*, with IL2-like alternatives, which induce a preferential signaling through the intermediate affinity IL-2 receptor (induce an IL2Rβγ-biased-IL2 signal). In other words, IL2-like alternatives directed to preferentially interact with the IL2-dimeric receptor (IL2Rβγ) and signal through it. Such alternatives include, but are not limited to the followings:
- Culturing the lymphoid cells with a soluble IL-2 mutein, which preferentially interact and signal though the intermediate affinity IL2R (IL2Rβγ).
- Genetically engineering of the lymphoid cells to secrete IL2 muteins, which preferentially signals though the intermediate affinity IL2R.
- Culturing the lymphoid cells in the presence of native IL-2, but:
   a) Adding a pharmacologic agent, which block IL2-IL2Rα interaction or,
   b) Genetically engineering the lymphoid cells to secrete a soluble protein, which block the IL2-IL2Rα interaction or,
   c) Genetically engineering the lymphoid cells to decrease the expression of the IL2Rα receptor subunit (CD25) at the cell surface or,
   d) Genetically engineering the lymphoid cells to increase the expression of the intermediate affinity IL-2 receptor at the cell surface.

Methods herein disclosed provide strategies for generating/expanding activated lymphoid cells with central memory phenotype, highly effective for tumor control when transferred in vivo. This invention represents a substantial improvement of the current strategies for ACT therapies.

Culture of lymphoid cells in accordance with the methods of the present invention could be performed using suitable culture medium and under suitable environmental conditions for the *in vitro* culture of immune cells. The lymphoid cell expansion could be done also in the presence of other cytokines, for instances IL12, IL17, IL15 and IL21. The lymphoid cell expansion could follow any of the reported protocols in the context of ACT. In the culture phases, where native IL2 is use in these protocols, any of the IL2 like alternatives described here in detail is used to substitute native IL2 provided signal.

Lymphoid cells in the methods could or could not have been further engineered to: express a CAR; express a TCR of desired specificity; express other receptors of interest in the cell membrane; Secrete different cytokines or soluble proteins of interest.

Lymphoid cells subject to the methods could be any of the followings: a mixture of T cells; purified CD4 of CD8 T cells, NK cells, NKT cells. Lymphoid cells could be obtained from PBMC, tumor draining lymph nodes or tumor infiltrates.

The terms "IL2 mutein", "noα-mutein", "noα-IL2-mutein", "IL-2 mutein", "partial agonist IL2", or "mutant IL2" are used herein interchangeably and refer to a mutated interleukin 2 protein, or a modified interleukin 2 protein, wherein the mutations induce the activation of the β/γ dimeric IL-2 receptor, preferentially interact with the IL2-dimeric receptor (IL2Rβγ), induce an IL2Rβγ-biased-IL2 signal, has a reduced affinity for IL2Rα, or has a higher affinity for the β/γ IL2 receptor. Non-limiting examples of these mutated or modified IL2 muteins are Seq. 1-7.

The terms "lymphocytes, or "lymphoid cell" are used herein interchangeably and refer to any cell that mediate the immunity of an animal including lymphocytes, lymphoblasts, and plasma cells. In some embodiments of the present invention, the terms refer to a class of white blood cells that bear variable cell-surface receptors for antigen and are responsible for adaptive immune responses. They can mediate innate and adaptive immune response. They can mediate humoral and cell-mediated immunity.

The strategies proposed in the present invention could be used for therapeutic purposes in ACT therapies for cancer treatment.

The present invention provides a method of treating a tumor in a subject in need thereof comprising administering to the subject the effective amount of a population of lymphocytes with central memory phenotype obtained by the methods disclosed herein. In certain embodiments, the tumor is a solid tumor (e.g., ovarian tumor, a melanoma, a lung tumor, a gastrointestinal tumor, a breast tumor). In certain embodiments, the tumor is a liquid tumor (e.g., a leukemia, or a lymphoma). In certain embodiments, the tumor expresses a mutation consistent with at least one peptide comprising a tumor antigen. In certain embodiments, the subject is human.

### Using engineered cytokines derived from IL2, which preferentially interact and therefore signal through the intermediate affinity IL2R (IL2Rβγ).

One embodiment of the present invention relates to specifically direct IL2 signaling through the intermediate affinity dimeric IL2 receptor-IL2Rβγ-during T cell activation and expansion protocols, using IL2 muteins. The IL2 muteins include, but are not limited to, variants with reduced IL2Rα interaction affinity, for example those described in US 9,206,243; variants with increased IL2Rβ interaction affinity, for example the superkine H9 described in Levin et al., 2012; or variants with the combination of both (less IL2Rα, more IL2Rβ interaction affinity). Lymphocytes are activated with these muteins combined with specific TCR signaling, CD3/CD28 activation and/or other survival cytokines. This IL2-like signal could be maintained all along the culture or could be used in specific phases of the culture, preferentially it should be provided during the very first activation moment. The muteins could be used in a range of concentrations form 1ng/ml to 1mg/ml.

The lymphoid cell expansion could follow any of the reported protocols in the context of ACT. But in the culture phases, where native IL2 is used in the original protocol, the IL2 mutein is added instead. For instance, it could be a protocol based only in culturing the cell with antibodies anti-CD3/CD28 and the IL2 mutein or it could combine the IL2 mutein with other cytokines as IL7, IL15, IL12 and IL21.

### Genetically engineering the lymphoid cells to secrete IL2 muteins, which preferentially signals though the intermediate affinity IL2R (IL2Rβγ).

Other embodiment of the present invention relates to genetically modifying the lymphoid cell to secrete any of the IL2 muteins referred in this invention. The modification of the lymphoid cells could be done with any of the well described transfections or transduction methods widely used in the context of ACT technology. For instance, this could be achieved using genome editing techniques or viral vectors to introduce on T cells a construct with the gen for the mutein. The constructs could also contain a reporter protein to easily track the transduction efficacy and an antibiotic resistance gen to enrich the transduced fraction.

Efficiency of transfection/transduction shall be high. At least high enough to grant that the lymphoid cells provide to them self an autocrine IL2Rβγ-biased-IL2 signal during the in vitro expansion. This particular procedure could grant to the lymphoid cells availability of the IL2Rβγ-biased-IL2 signal after the transference in vivo, further contributing to the cells persistence and anti-tumoral capacity.

The cell expansion of engineered lymphoid cells could follow any of the reported protocols in the context of ACT, but without adding any native IL2 to the culture. For instance, lymphoid cells could be activated with specific TCR signaling, CD3/CD28 activation alone and/or supplemented with survival cytokines as IL7, IL15 and IL21.

### Adding a pharmacological agent, which disrupt IL2-IL2Rα interaction, into lymphoid cells cultures.

Other embodiment of the present invention relates to different ways of disrupting pharmacologically the IL2/IL2Rα interaction while native IL2 is used in the *in vitro* lymphoid cells expansion for ACT. The addition of such pharmacological agent turns the signal of native IL2 into an IL2Rβγ-biased-IL2 signal, by blocking the access of IL2 to the high affinity trimeric IL2R.

The activation/expansion protocol could combine specific TCR signaling, CD3/CD28 activation and other cytokines of interest. The pharmacological agent and the IL2 should be used always together and added on culture at the same time. The resulting IL2Rβγ-biased-IL2 signal could be maintained all along the culture or could be used in specific phases of the culture, preferentially it should be provided during the very first activation period. The pharmacological agent should be used in excess to enforce its blocking capacity. The specific amounts shall be calibrated case by case, checking the capacity to block the binding of IL2 to CD25.

The pharmacologic agents, used to block the IL2-IL2Rα interaction, in the present invention includes, but are not limited to: An antibody or antibody fragment, which bind either to IL2Rα or to IL2; A peptide or a chemically defined small molecule, which bind either to IL2Rα or to IL2; A soluble form of the IL2Rα (CD25) or a modified variant of it.

In the case of pharmacological agents binding to IL2 they should be verified on their capacity to block only the interaction with IL2Rα, without affecting binding and signaling through IL2Rβγ. These agents could be added separately to the IL2 in the culture, but they could be also mixed with IL2 in advance to facilitate the formation of the desired complex. The lymphoid cell expansion in this embodiment could follow any of the reported protocols in the context of ACT. But in the culture phases, where native IL2 is used in the original protocol, the pharmacological agent is added too. For instance, it could be a protocol based only in culturing the cells with anti-CD3/CD28 mAbs and the IL2 +the blocking agent, or it could combine IL2+the blocking agent with other cytokines as IL7, IL15, IL12 and IL21

### Genetically engineering of lymphoid cells to secrete a soluble protein, which block interaction between IL2 and IL2Rα.

Other embodiment of the present invention relates to genetically modifying the lymphoid cell to secrete a soluble protein, which specifically block the interaction between IL2 and IL2R□. The modification of the lymphoid cells could be done with any of the well described transfections or transduction methods widely used in the context of ACT technology. For instance, this could be achieved using genome editing techniques or viral vectors to introduce on T cells a construct with the gen for the desired protein. Efficiency of transfection/transduction shall be high. At least high enough to grant that the lymphoid cells produce enough soluble protein as to significantly block IL2-IL2Rα interaction, granting for them self an IL2Rβγ-biased-IL2 signal during the in vitro expansion.

The protein, which could be used to block the IL2-IL2Rα interaction in the present invention includes, but are not limited to: an antibody or antibody fragment, which bind either to IL2Rα or to IL2; A soluble form of the IL2Rα (CD25) or any modified variant.

The expansion of lymphoid cells in this embodiment could follow any of the reported protocols in the context of ACT. In this case without any variation, but those strictly necessary to engineered the cells. For instance, in culturing the cell with antibodies anti-CD3/CD28 and IL2 or it could combine IL2 with other cytokines as IL7, IL15, IL12 and IL21.

### Genetically engineering the lymphoid cells to decrease the expression of the IL2Rα receptor subunit (CD25) at the cell surface.

Another embodiment of the present invention relates to the genetic modification of the lymphoid cells to reduce the expression of IL2R□ at the cell surface. For cell engineering several strategies could be used, including but not limited to, interference RNA, genome-editing techniques and knockout strategies The modification of the Lymphoid cells could be done with any of the well described transfections or transduction methods widely used in the context of ACT technology. For instance, the reduction or total suppression of CD25 expression on lymphoid cells could be achieved using viral vectors to introduce on the cells a construct with shRNA targeting the gen for CD25.

The expansion of lymphoid cells in this embodiment could follow any of the reported protocols in the context of ACT. In this case without any variation, but those strictly necessary to genetically engineered the cells. For instance, the engineered cells could be cultured with antibodies anti-CD3/CD28 and IL2 or it could combine IL2 with other cytokines as IL7, IL15, IL12 and IL21.

### Genetically engineering the lymphoid cells to increase the expression of the intermediate affinity IL-2 receptor at the cell surface

Another embodiment of the present invention relates to genetically modifying the lymphoid cells to increase the expression of the dimeric receptor IL2Rβγ on the cell surface. This could be achieved using viral vectors to introduce on T cells constructs with CD122 (IL2Rβ chain) gen or CD132 (IL2Rγ chain) gen under constitutive-expression promoters. Alternatively, for this strategy T cells can be modified to express mutated CD122 (IL2Rβ chain) with increased IL2-affinity instead of the native CD122. This could be achieved using genome editing techniques or viral vectors to introduce on lymphoid cells a construct with the gen for the mutated or wild type CD122.

For the described strategies, the constructs could also contain a reporter protein to easy track the transduction efficacy and an antibiotic resistance gen to enrich the transduced fraction.

The expansion of lymphoid cells in this embodiment could follow any of the reported protocols in the context of ACT. In this case without any variation, but those strictly necessary to genetically engineered the cells. For instance, the engineered cells could be cultured with antibodies anti-CD3/CD28 and IL2 or it could combine IL2 with other cytokines as IL7, IL15, IL12 and IL21.

### Methods of Treatment

In a related aspect, disclosed herein is a method for treating a tumor in a subject in need thereof comprising administering to the subject the effective amount of a population of lymphocytes produced by the methods disclosed herein. In certain embodiments the tumors are solid tumors. In certain embodiments, the tumors are liquid tumors (e.g. blood cancers).

### BRIEF DESCRIPTION OF THE FIGURES

**Figure 1****.** Comparison of T cells phenotype obtaining when cultured with native IL2 or with the IL2 mutein that preferential signaling through the IL2Rβγ. A) Culture survival, B) Percentage of memory and effector cells.
**Figure 2****.** Comparison of the phenotypes recovered on T cells when cultured with native IL2 or IL2-mutein that preferentially signals through the intermediate affinity receptor IL2Rβγ in a broad range of doses. A) Culture survival, B) Percentage of PD1 positive cells and C) Percentage of cells with Central memory-like phenotype.
**Figure 3****.** Comparison of the phenotypes recovered on T cells when cultured with native IL2 or IL2-mutein that preferentially signals through the intermediate affinity receptor IL2Rβγ combined with IL7 and IL15 cytokines A) Culture survival, B) Percentage of memory and effector cells.
**Figure 4****.** Comparison of the phenotype recovered on T cells when cultured with native IL2 or IL2-mutein that preferentially signals through the intermediate affinity receptor IL2Rβγ, in a broad range of doses, combined with IL7 and IL15. A) Culture survival, B) Percentage of PD1 positive cells and C) Percentage of cells with central memory-like phenotype.
**Figure 5****.** Comparison of the phenotypes recovered on T cells when stimulated with IL2 or IL2-mutein only or with IL2 or IL2-mutein combined with IL7 and IL15.
**Figure 6A****.** Histograms showing the percentage of eGFP positive cells, which measure the efficiency of transduction for IL2 or IL2 mutein in T cells.
**Figure 6B****.** Comparison of the percentage of cells with central memory and effector memory phenotype recovered in cultures when T lymphocytes were transduced to produce native IL2 or IL2-mutein.
**Figure 7A****.** Histograms showing the efficiency of CD25 downregulation after transduction with different shRNA constructs.
**Figure 7B****.** Percentage of cells with central memory and effector memory phenotype when T lymphocytes have reduced expression of CD25.
**Figure 8****.** Comparison of the effect of provide an IL2Rβγ-biased-IL2 signaling when employed an anti-CD25 antibody on CD8+ T cells A) Culture survival and B) Percentage of memory and effector cells.
**Figure 9****.** Functional capacity of CD8+ T cells with IL2 alone, IL2+IL15+IL7, IL2-mutein alone or IL2-mutein+IL15+IL7. A) Granzyme β production by OTI CD8+ T cells re-stimulated with the peptide SIINFEKL, B) Tumor killing capacity against B16-OVA cells of OT1 cells cultured with IL2, IL2-mutein alone or combined with IL7 and IL15.
**Figure 10****.** Measuring of tumor volume in MB16OVA tumor bearing animals that received OT1 cells cultured with native IL2 or IL2 mutein.
**Figure 11****.** Comparison of antitumor in vivo effect of OT1 T cells transduced to produce native IL2 or IL2-mutein that preferentially signals through the intermediate affinity receptor IL2Rβγ. A) Tumor volume, B) Survival over time.
**Figure 12**. Characterization of CD8+ TILs before and after culturing with IL2 or IL2 mutein: A) Expansion level, B) Ki-67 expression percentage by TILs and C) Distribution of naïve T cells (CD62L+CD44-), central memory (CD62L+CD44+) and (CD62L-CD44+).
**Figure 13****.** Characterization of CD8+ TILs before and after culturing with IL2 or IL2 mutein: A) Percentage of expression of inhibitory markers and B) IFNγ, TNFα and Granzyme β.
**Figure 14****.** Expansion of NK cells in TILs cultured with IL2 or IL2 mutein: A) Percentage of NK cells and B) NK cells number.

### EXAMPLES

### Example 1. Substitution of native IL2 by IL2-mutein, which preferentially signals through the IL2Rβγ, preserves lymphocyte viability and confers Central memory-like phenotype.

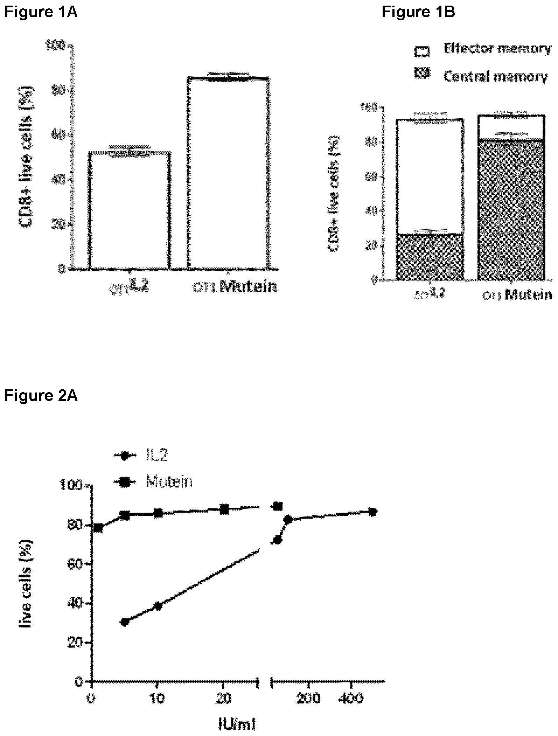

CD8+ T cells are isolated from naïve OT1 mouse spleen. AntiCD3/antiCD28 coated beads are used as TCR stimulation for seven days, together with native IL2 or the IL2 mutein for 10 days , the last one obtained in the Center of Molecular Immunology (batch 1201602) which and divulged in SEQ ID NO 6 of patent US 9,206,243. Every two days culture is expanded to keep the density at 1×10⁶ and the cytokines are added with fresh media. The successful expansion of a large numbers of T cells is achieved in both culture conditions and similar fold-increase in cell number is observed. Figure 1A shows that the viability in culture is improved when using the IL2 mutein in comparison to the native IL2 (p< 0.0001). Additionally, the quantity of memory cells and the expression of inhibitory markers was measured by flow cytometry. A significantly high percentage of cells with central memory-like phenotype (defined by CD62L+/CD44+) was observed in cells in when using IL2-mutein in comparison to native IL2 (Figure 1B). Phenotypic analysis of PD-1, Lag-3, Tim-3, and Tigit markers showed that IL2-mutein activated cells repressed the expression of the immune checkpoint ligands (PD-1, Lag-3, Tim-3, Tigit) (Table 1).

**Table 1. Exhaustion markers expression (percent of positive cells) when native IL2 or IL2-mutein is used for in vitro T cells activation/expansion.**

| **Marker** | **native IL-2** | **IL-2 mutein** |
|---|---|---|
| **PD1** | 2.61 | 0.38 |
| **TIM3** | 17.0 | 0.42 |
| **Lag3** | 79.9 | 15.9 |
| **Klrg1** | 12.4 | 0.06 |

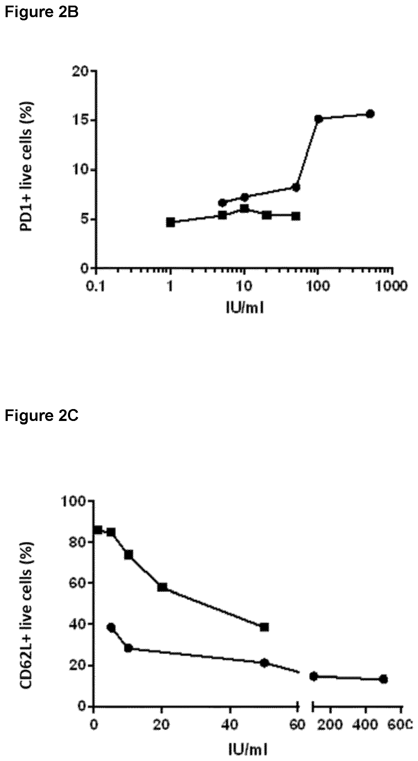

The described effect in terms of viability (Figure 2A), PD1 expression (Figure 2B) and CD62L expression (Figure 2C) is consistent in a broad range of doses for both the native IL2 and the IL2-mutein.

Taken together these results indicate that the substitution of native IL2 by IL2-mutein with preferential IL2Rβγ stimulation, promotes a central memory phenotype and not the well-described effector phenotype induced by native IL2. IL2-mutein more than native IL2 confers potential for lymphoid recirculation (more CD62L expression) and resistance to exhaustion (less PD1 expression), a more desired phenotype for *in vivo* transference.

### Example 2. Substitution of native IL2 by IL2-mutein, which preferentially signal through the IL2Rβγ, favors central memory phenotype in combined culture protocols with IL7 and IL15.

As previously described by other authors, the combination of IL7 and IL15 with IL2 improves T cells fitness when compared with IL2 alone (Redeker and Arens, 2016, Front Immunol. 6(7): 345). In our experiment, native IL2 or IL2-mutein were combined in culture with IL7 and IL15 for OT-I T cells activation/expansion protocol.

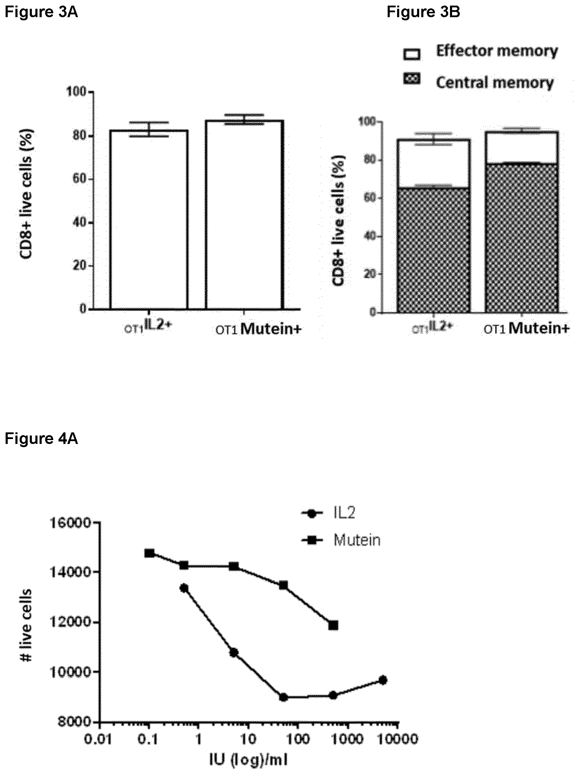

In this strategy, the same TCR stimulation described in Example 1 was used. Native IL2 or IL2-mutein is added at the beginning of the culture until day 5^{th}, then the cells are kept in IL7/IL15 for 10 days. In this scenario, both culture conditions give similar cell density in culture, high viability (Figure 3A), and favors the prevalence of memory rather than effector cells. However, the use of IL2-mutein showed higher proportion of central memory cells in culture compared to native IL2 (p< 0.0015) as shown in Figure 3B.

At the same time, the cells cultured with the mutein showed a lower expression of exhaustion markers as shown in Table 2.

**Table 2. Exhaustion markers expression (percent of positive cells) when native IL2 or IL2-mutein is used for in vitro T cells activation/expansion combined with IL7 and IL15.**

| **Marker** | **native IL-2** | **IL-2 mutein** |
|---|---|---|
| PD1 | 1.69 | 0.48 |
| TIM3 | 10.3 | 0.18 |
| Lag3 | 84.3 | 21.1 |
| Klrg1 | 6.07 | 0.05 |

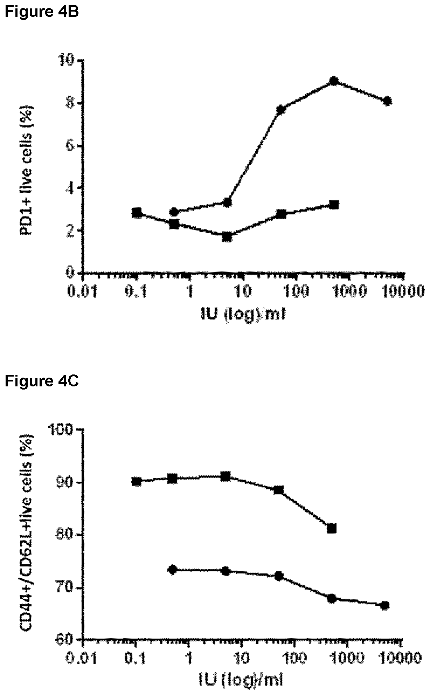

The evaluation of different concentrations in culture, for both the native IL2 and the IL2-mutein combined with IL7/IL15, demonstrates that the described effect is consistent in a broad range of doses, in terms of viability (Figure 4A), PD1 expression (Figure 4B) and central memory-like phenotype (Figure 4C).

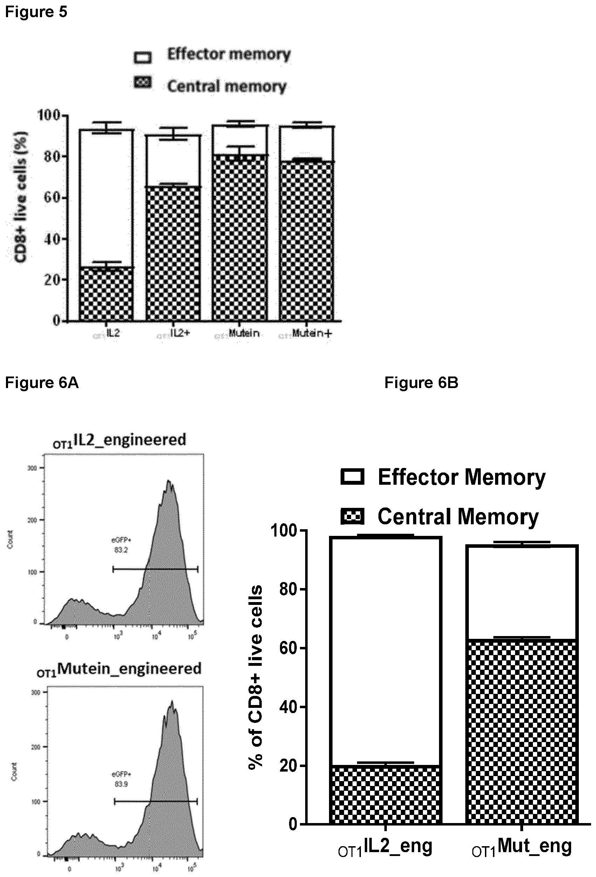

Although the combination of native IL2 with IL7 and IL15 improves culture conditions when compared with native IL2 alone, no improvement is observed for the IL2-mutein when using combined protocols, since IL2-mutein alone is highly effective in preserving T cells viability and inducing central memory phenotype. Additionally, IL2-mutein alone is as effective as the combination of IL2, IL7 and IL15 in inducing central memory phenotype-like on activated T cells (Figure 5).

### Example 3. Genetically engineered T cells that produce IL2-mutein, which preferentially signal through the IL2Rβγ, acquire a central memory phenotype.

In order to generate a continued cytokine support source both *in vitro* and *in vivo,* we genetically engineered T cells to produce the IL2-mutein that preferentially activate the intermediate affinity IL2 receptor. Retroviral constructs encoding the enhanced green fluorescent protein (eGFP) and the native IL2 or IL2-mutein are used. The transduction procedure is initiated by stimulating freshly isolated naïve OT-I T cells with anti-CD3/anti-CD28 coated beads and native IL2 or IL2-mutein. Concentrated retroviral vector supernatant is added at 24 and 48 hours to a retronectin coated-plate with the cells. After transduction, TCR stimulation with anti-CD3/anti-CD28 coated-beads is maintained for seven days together with IL7 and IL15 for 10 days. The transduction efficiency was confirmed by eGFP expression and was higher than 80% for both constructs (Figure 6A). The secreted molecule was detected by ELISA (native IL2 or IL2-mutein).

In a similar manner to that observed when OT1 T cells were cultured with the soluble IL2 mutein, the cells engineered to produce IL2-mutein had the memory phenotype in a higher percentage when compared to the cells engineered to produce the native IL2 which were mainly effector cells (p< 0.0001) (Figure 6B). Also, the cells engineered to produce IL2-mutein had less exhaustion markers expression compared to the cells engineered to produce the native IL2 (Table 3).

**Table 3. Exhaustion markers expression (percent of positive cells) when T cells are engineered to produce native IL2 or IL2-mutein**

| **Marker** | **native IL-2** | **IL-2 mutein** |
|---|---|---|
| PD1 | 3.02 | 0.67 |
| TIM3 | 17.0 | 0.35 |
| Lag3 | 80.1 | 36.2 |

Taken together these results confirms the hypothesis that stimulation through IL2Rβγ with the IL2-mutein, favors central memory differentiation with this new culture method when the IL2-like signaling is constant and sustained.

### Example 4. Genetically engineering to downregulate CD25 expression, favors a central memory phenotype on T cells expanded with native IL2.

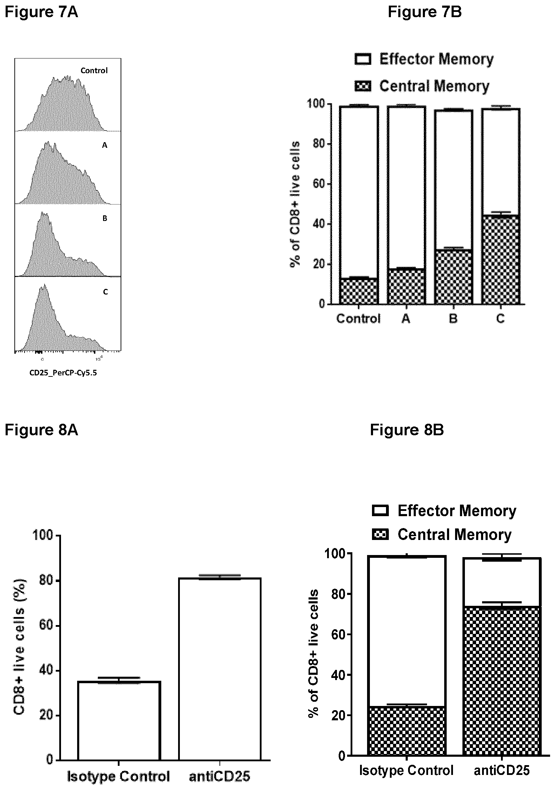

Vectors encoding for eGFP and a small hairpin RNA (shRNA) targeting *il2ra* were used, with the aim of blocking the expression of IL2 alpha chain gen (CD25). The transduction procedure is initiated by stimulating freshly isolated naïve OT1 T cells with anti-CD3/anti-CD28 coated beads and native IL2. Three different constructions for shRNA were used, obtaining variable knockdown efficiencies based on surface expression of CD25. Transduction with lentivirus containing scramble shRNA is used as control. After two rounds of transduction, cells are maintained in culture for 5 days with native IL2 (50 U/ml). The reduction on CD25 surface expression after transduction with the different constructs was evaluated by Flow Cytometry (Figure 7A). The percentage of central memory cells after five days of native IL2 stimulation is superior when better CD25 downregulation is achieved (Figure 7B).

Downregulation of CD25 on T cells provides a scenario for preferentially stimulation of the intermediate affinity receptor IL2Rβγ using in culture the native IL2. Under these conditions, the differentiation of CD8+ T to the central memory-like phenotype is favored.

### Example 5. Central memory phenotype is obtained when cells are stimulated with native IL2 but CD25 interaction is blocked with a pharmaceutical agent simultaneously added on the culture.

In another attempt to preferentially direct native IL2 signaling through intermediate affinity receptor IL2Rβγ, a pharmaceutical agent is added on culture together with native IL2 during *in vitro* T cells activation. The pharmacological agent is added at high concentration to guarantee the blocking effect. OT-I T cells are cultured with anti-CD3/anti-CD28 coated beads, 50 IU/ml of IL2 and 10 µg/ml of anti-CD25 monoclonal antibody (BioLegend clone 3C7). The anti-CD25 is added since day 0 and is renewed when adding fresh medium with native IL2 every two days. An antibody with irrelevant specificity is used as isotype control. Phenotyping of the cells after 10 days of culture revealed that the addition of anti-CD25 during native IL2 activation, increases the cell viability (Figure 8A) and the central memory-like population (Figure 8B) (p< 0.0001) when compared with the isotype control. Additionally, when native IL2 interaction with CD25 is disrupted with the antibody, less expression of exhaustion markers is obtained (Table 4).

**Table 4. Exhaustion markers expression (percent of positive cells) when T cells are stimulated with native IL2 but interaction with CD25 is disrupted with an antiCD25 antibody**

| **Marker** | **Isotype Control** | **Anti-CD25** |
|---|---|---|
| PD1 | 4.12 | 3.33 |
| TIM3 | 18.5 | 0.54 |
| Lag3 | 80.2 | 21.3 |

Together, these results suggest that directing native IL2 signaling through IL2Rβγ by impairment of the interaction with CD25, the cells are directed to a central memory phenotype, which is more suitable for adoptive cell therapies.

### Example 6. OT-I T cells which receive preferential activation through dimeric receptor IL2Rβγ, are polifunctional and show efficient T cell antigen-specific cytotoxicity in vitro.

In order to assess the functional capacity of the central memory T cells, activated through the IL2Rβγ, a cytotoxicity assay was performed. OT1 cells activated as described in Examples 1, 3 were stimulated for 16 hours with the cognate peptide SIINFEKL, and flow cytometry analysis was performed to determine the production of Granzyme β. In order to evaluate the killing capacity of the OT-I T cells, the cells were co-cultured with B16 melanoma cells transduced to express the OVA nominal antigen in the surface (B16-OVA) at the 1:1 proportion. Non transduced B16 melanoma cells were used as control. IncuCyteCytotox reagent was added and the killing capacity was evaluated each two hours during 60 hours. The lysis of the target cells was antigen specific, due to no lysis was observed in the controls cells.

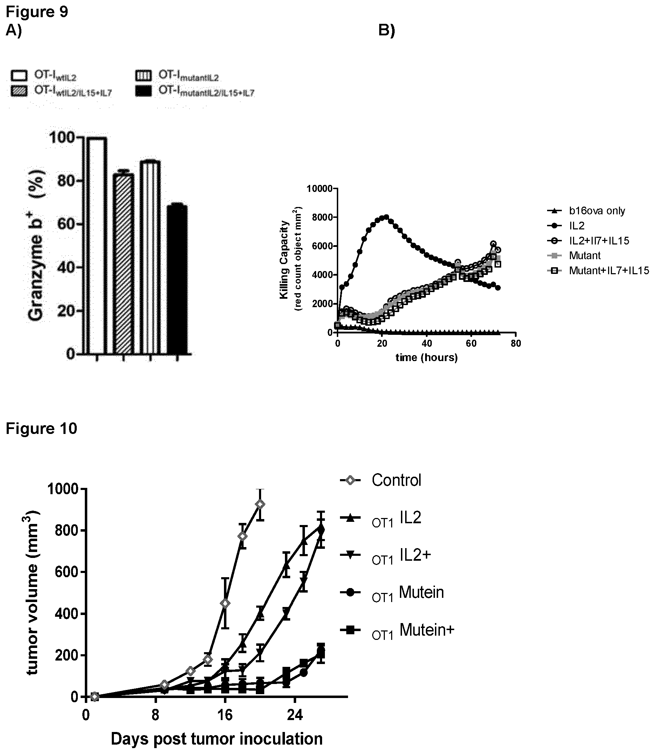

OT1 cells activated with the IL2 mutein that preferentially signaling through IL2Rβγ showed an *ex vivo* effective direct lysis activity, showing when signal is given through the IL2Rβγ the cells are differentiated into potent killing cells. (Figure 9A and B)

### Example 7. T cells, which receive preferential activation through dimeric receptor IL2Rβγ, show better antitumor activity when used for ACT in a melanoma model.

The OT1 model together with B16/OVA tumor cell line is one of the most used models and represents a relevant preclinical approximation of ACT. C57BL/6 recipient mice received a subcutaneous injection of 1×10⁶ OVA-expressing melanoma cells on day 0 and ten days after tumor cells inoculation; mice were sub-lethally irradiated (5Gy).
Group 1: received no treatment (control group).
Group 2: received OT1 T cells activated with native IL2 (as in Example 1).
Group 3: received OT1 T cells activated with IL2-mutein (as in Example 1).
Group 4: received OT1 T cells activated with native IL2+IL7+IL15 (as in Example 2).

As shown in Figure 10, ACT with cells primed with IL2-mutein better controlled tumor growth compared to native IL2-activated cells. Together these results indicates that the initially induced memory phenotype with low exhaustion markers expression obtained with IL2-mutein activation was an effective combination for tumor control *in vivo.*

### Example 8. T cells engineered to secrete IL2-mutein, which preferentially signal through the IL2Rβγ, showed robust antitumor activity when used for ACT.

In an effort to improve the effectiveness of ACT, we genetically engineered T cells to produce the IL2-mutein. The insertion of the gen allows for continued and sustained IL2-mutein signal on T cells, not just *in vitro* but *in vivo* as well. C57BL/6 recipient mice received a subcutaneous injection of 1×10⁶ OVA-expressing melanoma cells on day 0 and ten days after tumor cells inoculation; mice were sub-lethally irradiated (5Gy). The animals were divided in three groups of treatment and on days 11 and 15 they received an intravenous transference of 1×10⁶T cells from OT1 mice as follow:
Group 1: received no treatment (control).
Group 2: received native IL2_engineered OT1 T cells (as the transduction protocol described in Example 3)
Group 3: received IL2-mutein_engineered OT1 T cells (as the transduction protocol described in Example 3).

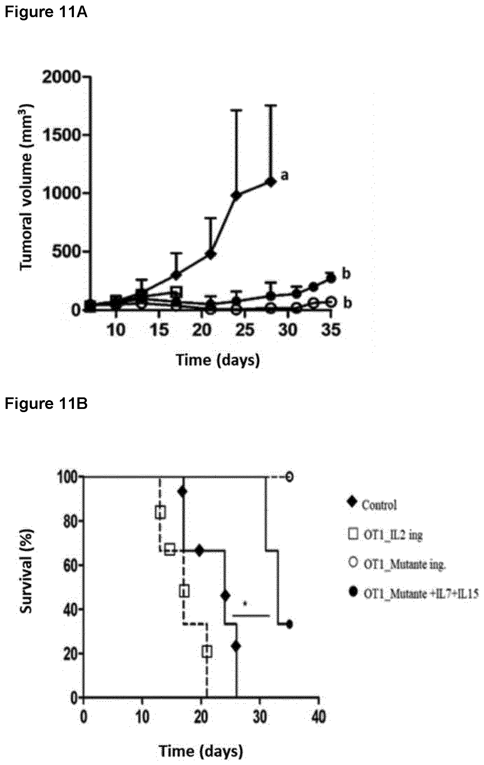

As shown in Figure 11, the mice receiving OT1 T cells engineered for IL2-mutein production, experienced a good control of the stablished tumors (Figure 11A) and a higher survival (Figure 11B).

### Example 9 Generation of TILs using IL2-mutein.

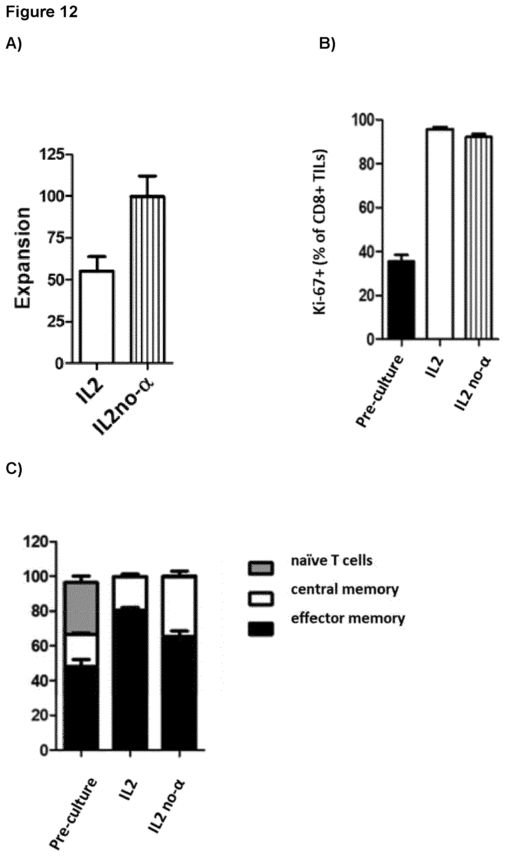

Since ACT therapies can be performed using TILs, we evaluated the effects of IL2-mutein on the TILs expansion. To study the role of the activation of the β/γ dimeric IL2 receptor in tumor-infiltrating lymphocytes, MC38 tumors were isolated from C57BL/6 mice between days 19-21 following tumor cell inoculation. Tumors were mechanically dissociated and were cultured in presence of IL2 or IL2-mutein for 14-16 days. We found that TILs expansion was higher when IL2-mutein was used in culturing conditions. Consistently, IL2-mutein increased the proliferation rate of TILs in terms of ki67 marker (Figure 12A and B). Furthermore, TILs cultured *ex vivo* with IL2-mutein showed a significant expansion of central memory-like T cells as compared to the controls (Figure 12C). Overall, these data show that IL2-mutein induces CD8+ TILs expansion favoring central memory differentiation phenotype.

### Example 10. TILs expanded with mutant IL-2 are polyfunctional and show reduction of activation and exhaustion markers.

TILs obtained with the methodology of Example 9 were characterized functional and phenotypically before the expansion, they were stimulated during 4 hours in presence of anti-CD3 and anti-CD2 antibodies.

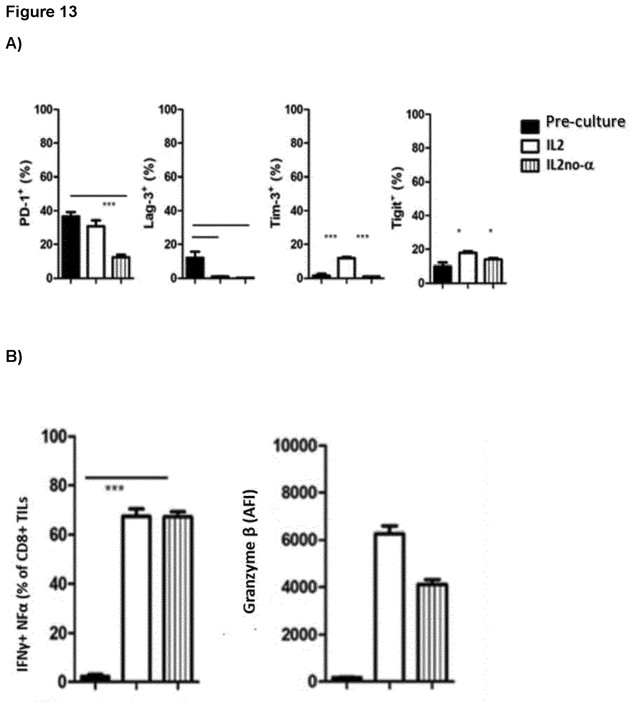

We found that inhibitory receptors PD-1, Lag-3, and Tim-3 were drastically reduced in TILs expanded with IL2-mutein compared with TILs expanded with native IL2 (Figure 13A). TILs were also analyzed for their functionality. (Figure 13B). Collectively, these data show the ability of IL2-mutein to expand TILs and enrich them for central memory-like phenotype without compromising their functionality.

### Example 11. Generation of NK cells from TILs using mutant IL-2.

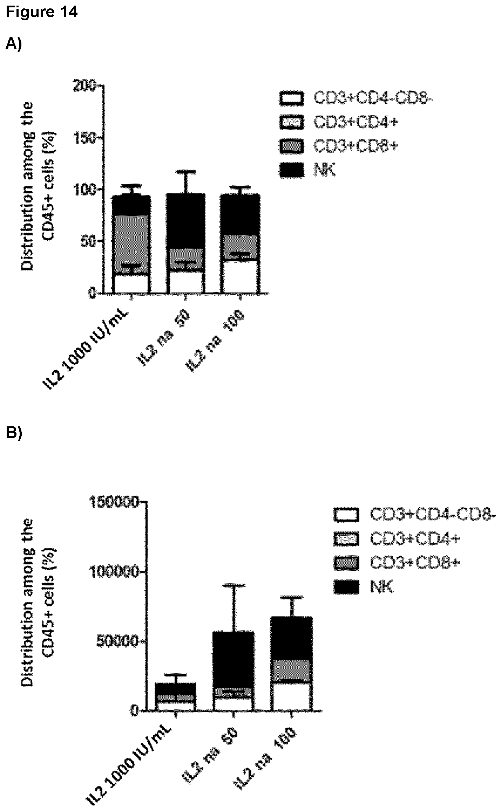

To study the role of the activation of the β/γ dimeric IL2 receptor in the proportion of NK cells among the total expanded TILs, MC38 tumors were isolated from C57BL/6 mice between days 19-21 following tumor cell inoculation. Tumors were mechanically dissociated and were cultured in presence of native IL2 or IL2-mutein for 14-16 days. TILs cultured *ex vivo* with IL2-mutein showed a significant expansion of NK cells as compared to the native IL2, in terms of proportion form the total (Figure 14A) and in terms of absolute numbers (Figure 14B).

## Claims

1. A method for the differentiation and expansion of T lymphocytes and NK cells with a central memory phenotype useful in adoptive cell transfer therapies comprising three stages:
i) extraction of T lymphocytes and/or NK cells from a subject,
ii) *in vitro* expansion and differentiation of T lymphocytes and/or NK cells, with a strategy that guarantees a preferential signaling through the intermediate affinity IL2 receptor (IL2Rβγ), optionally said T lymphocytes and/or NK cells can be engineered, and
iii) transferring the activated cells to a subject.

2. The method according to claim 1 wherein the strategy for inducing a preferential signaling through the intermediate affinity IL2 receptor (IL2Rβγ) is selected from the group comprising:
- culturing the T lymphocytes and NK cells with a soluble IL2 mutein that has the property of preferentially signaling through IL2Rβγ,
- genetically modifying the T lymphocytes and NK cells to secrete an IL2 mutein that has the property of preferentially signaling through IL2Rβγ,
- culturing the T lymphocytes and NK cells in the presence of native IL2 and a pharmacological agent which blocks the IL2-IL2Rα (CD25) interaction,
- culturing the T lymphocytes and NK cells in the presence of native IL2 and genetically modifying the T lymphocytes and NK cells to secrete a soluble protein that blocks the IL2-IL2Rα interaction,
- culturing the T lymphocytes and NK cells in the presence of native IL2 and genetically modifying the T lymphocytes and NK cells to decrease the expression of IL2Rα at the cell surface,
- culturing the T lymphocytes and NK cells in the presence of native IL2 and genetically modifying the T lymphocytes and NK cells to increase the expression of IL2Rβγ.

3. The method according to claim 2 wherein the IL2 mutein is selected from the group comprising of:
- SEQ ID NO. 1,
- SEQ ID NO. 2,
- SEQ ID NO. 3,
- SEQ ID NO. 4,
- SEQ ID NO. 5,
- SEQ ID NO. 6 and
- SEQ ID NO. 7.

4. The method according to claim 2 wherein the pharmacological agent which blocks interaction of IL2 with the α chain of its receptor is selected from the group comprising of:
- an antibody or antibody fragment which binds to IL2 or to the α chain of the IL2 receptor
- a peptide or a chemically defined small molecule which binds to IL2 or to the α chain of the IL2 receptor, and
- a soluble form of the α chain of the IL2 receptor or a modified variant of it.

5. The method according to claim 1 wherein stage (iii) can be performed on the same subject donating the cells or in a different subject.

6. The method according to claim 2 wherein the signaling through IL2Rβγ may occur in different moments of cultivation of the cells or of maintenance *in vitro* and *in vivo.*

7. The method according to claim 1-6 to prolong the persistence of the transferred cells and a higher antitumor effect *in vitro* and *in vivo.*

8. The method according to claim 1-6 in combination with other cytokines selected from the group comprising:
- IL12,
- IL17,
- IL15, and
- IL21.

9. The method according to claim 1 wherein the T lymphocytes and NK cells can be any of the followings: a mixture of T cells; purified CD4 or CD8 T cells; NK cells; or NKT cells.

10. The method according to claim 1 wherein the T lymphocytes and NK cells in stage i can be obtained from peripheral blood mononuclear cells, tumor draining lymph nodes or tumor infiltrates.

11. The method according to claim 1 wherein the T lymphocytes and NK cells can further be engineered to express a CAR, a TCR of desired specificity, other receptors of interest in the cell membrane, secrete different cytokines or soluble proteins of interest.

12. Use of the method of any of the claims 1-6 for adoptive cell transfer therapy.

13. Use according to claim 9 for obtaining tumor-infiltrating lymphocytes or chimeric T cell receptors.

14. Use according to claims 11-12 for the treatment of cancer.

15. The cells obtained by the method of claims 1-6.

16. A method for enrichment and expansion *ex vivo* of lymphocytes with a memory phenotype comprising:
a) Obtaining a population of lymphocytes from a subject.
b) Expanding the lymphocytes by culturing the cells under conditions that activate in said lymphocytes the dimeric IL2 receptor.

17. The method of claim 16, wherein the step (b) is performed by using at least one IL2 mutein.

18. The method of claim 16, wherein the step (b) is performed by regulating the expression of at least one subunit of the IL-2 receptor.

19. The method of claim 18, wherein the subunit of the IL2 receptor is the alpha (CD25), beta, or gamma subunit.

20. The method of claim 16, wherein the step (b) is performed by inhibiting the interaction of IL-2 receptor with its cognate protein.

21. The method of claim 20, wherein the inhibition of the interaction of IL2 receptor with its cognate protein occurs by downregulating the alpha subunit of IL2 receptor, or by upregulating the beta and gamma subunits.

22. The method of claim 20, wherein the inhibition of the interaction of IL2 receptor with its cognate protein occurs by incubating the cells with an anti-CD25 antibody.

23. The method of claim 16, wherein the step (b) is performed by transducing lymphocytes with a nucleic acid coding for the expression and secretion of the IL2 mutein.

24. The pharmaceutical composition obtained by any of claims 16-23.

25. A method of treating a cancer in a subject in need thereof, the method comprising administering lymphocytes with a central memory phenotype comprising:
a) Obtaining a population of lymphocytes from a subject;
b) Expanding the lymphocytes by cultivating the cells under conditions that activate in said lymphocytes the dimeric IL2 receptor; and
c) Administering a therapeutically effective dose of the lymphocytes from step (b) to the subject.

26. The method of claim 25, wherein activating the βγ dimeric IL2 receptor comprises incubating said lymphocytes with an IL2 mutein.

27. The method of claim 25, wherein activating the βγ dimeric IL2 receptor comprises introducing in said lymphocytes a nucleotide sequence encoding an IL2 mutein.

28. The method of claim 25, wherein activating the βγ dimeric IL2 receptor comprises introducing a nucleotide sequence to upregulate the expression of the subunits β and γ of the IL2 receptor in said lymphocytes and incubating said lymphocytes with wild-type IL2.

29. The method of claim 25, wherein activating the βγ dimeric IL2 receptor comprises introducing a nucleotide sequence to downregulate the expression of the alpha subunit of the IL2 receptor in said lymphocytes and incubating said lymphocytes with wild-type IL2.

30. The method of claim 25, wherein activating the βγ dimeric IL2 receptor comprises incubating said lymphocytes with wild-type IL2 and anti-CD25 antibodies.
